# EUROPEAN PATENT SPECIFICATION

(11) **EP 2 674 500 B1**
(45) Date of publication and mention of the grant of the patent: **14.06.2023**
(21) Application number: 12744530.2
(22) Date of filing: 31.01.2012
(51) Int. Cl.: C12P 19/12, C07H 3/04

(54) **METHOD FOR PRODUCING SOLID MATTER FROM SACCHARIDE SOLUTION, AND SOLID MATTER**
VERFAHREN ZUR HERSTELLUNG EINES FESTSTOFFS AUS EINER SACCHARIDLÖSUNG UND FESTSTOFF
PROCÉDÉ POUR PRODUIRE UNE MATIÈRE SOLIDE À PARTIR D'UNE SOLUTION DE SACCHARIDE, ET MATIÈRE SOLIDE

(30) Priority: 10.02.2011 JP 2011027216; 18.11.2011 JP 2011252794
(43) Date of publication of application: 18.12.2013
(73) Proprietor: Mitsui Sugar Co., Ltd., Chuo-ku Tokyo 103-8423 (JP)
(72) Inventor: OKUNO, Masahiro, Chigasaki-shi, Kanagawa 253-0042 (JP); MIYASAKA, Kiyoaki, Chigasaki-shi, Kanagawa 253-0042 (JP)
(74) Representative: Cabinet Nony
(86) International application number: PCT/JP2012/052070
(87) International publication number: WO 2012/108293

(56) References cited:
- EP-A1- 0 028 900
- EP-A1- 1 550 666
- JP-A- 2005 192 449
- JP-A- 2009 536 168
- US-A1- 2009 209 662
- Ana Lucia Leite Moraes: "PRODUÇÃO DE ISOMALTULOSE A PARTIR DA TRANSFORMAÇÃO ENZIMÁTICA DA SACAROSE, UTILIZANDO-SE Erwinia sp D12 IMOBILIZADA COM ALGINATO DE CÁLCIO (PRODUCTION OF ISOMALTULOSE FROM ENZYMATIC TRANSFORMATION OF SUCROSE, USING Erwinia sp D12 IMMOBILIZED WITH CALCIUM ALGINATE.)", Cienc. Tecnol, Aliment., Campinas 25(1), 1 January 2005 (2005-01-01), pages 95-102, XP055256921, Retrieved from the Internet: URL:http://www.scielo.br/pdf/cta/v25n1/a15 v25n1.pdf [retrieved on 2016-03-09]
- DATABASE WPI Week 198144 Thomson Scientific, London, GB; AN 1981-80443D XP002755301, & JP S56 117796 A (MITSUI SEITO KK) 16 September 1981 (1981-09-16)
- DATABASE WPI Week 199149 Thomson Scientific, London, GB; AN 1991-358274 XP002755302, & JP H03 240463 A (MITSUI SUGAR CO LTD) 25 October 1991 (1991-10-25)

## Description

### FIELD OF THE INVENTION

The present invention relates to a method for producing a solid material from a saccharide, especially from a saccharide liquid comprising isomaltulose, allowing an alpha-glucosyltransferase to act on a sucrose liquid, wherein the solid material comprises isomaltulose in an amount of 70 to 90 mass% and trehalulose of an amount of 8 to 25 mass%, and is an aggregate of isomaltulose crystals and non-crystalline saccharide liquid which comprises trehalulose.

### BACKGROUND OF THE INVENTION

Isomaltulose is a disaccharide which is obtained by allowing an alpha-glucosyltransferase which is an enzyme produced by Protaminobacter rubrum, Serratia plymuthica, Erwinia rhapontici, or Klebsiella sp., to act on sucrose so that an alph-1,2 bond in sucrose is changed into an alpha-1,6 bond.

Saccharide composition of the saccharide liquid obtained by allowing the enzyme to act on a sucrose liquid is isomaltulose of 60 to 90 mass%, trehalulose of 5 to 35 mass%, glucose of 0.2 to 5 mass%, and fructose of 0.2 to 5 mass%. The saccharide liquid is concentrated and made to generate isomaltulose crystals (crystallization step) and, then, the generated crystals are collected by centrifugation (centrifugation step). In this way, crystallized isomaltulose is obtained by subjecting the saccharide liquid to the crystallization step and the centrifugation step. Crystallized isomaltulose is sold as crystalline Palatinose(trademark) IC (Mitsui Sugar Co., Ltd., an isomaltulose purity of 99.0% or more) . Meanwhile, saccharide composition of a syrup obtained in the centrifugation step is trehalulose of 53 to 59% and isomaltulose of 11 to 17%. This syrup is sold as Palatinose(trademark) syrup-ISN (Mitsui Sugar Co., Ltd.).

A solubility of isomaltulose in water is low and, therefore, isomaltulose crystals are easily generated. On the other hand, trehalulose does not crystallize and remains as liquid. Therefore, the centrifugation step is essential for removing trehalulose to sell the separated isomaltulose as a solid product or a powdery product. In the centrifugation step, the crystallized isomaltulose and the syrup are obtained in a certain proportion. However, a supply for the syrup does not balance sometimes with a demand for it and, therefore, the syrup is left over.

The following Patent Literature Nos.1 to 9 describe methods for solidifying saccharides. Patent Literature Nos.1, 2 and 9 describe methods for solidifying Palatinose. Patent Literature Nos.3 to 7 describe methods for solidifying saccharides other than Palatinose. Patent Literature No. 8 describes a method for solidifying hydrogenated Palatinose.

Patent Literature No.1 describes a method for producing a saccharide composition, characterized in that, in converting sucrose to Palatinose by a bacterial enzyme which converts sucrose to Palatinose, the contents of glucose and fructose which are by-products in the conversion are controlled by changing a temperature, and saccharides generated in the conversion are solidified all together (claim 1). As a means for the solidification, a solidification and pulverization method is mentioned, where a saccharide liquid is highly concentrated to form a viscous massecuite-like liquid and, then, the liquid is cooled, solidified, and dried by warm air while being crushed, and pulverized (page 2, lower left column, lines 10 to 17).

Patent Literature No.2 describes a method for producing amorphous Palatinose (claim 1). The method is characterized in that Palatinose crystals and water of 3 to 9% based on the Palatinose crystals are fed to a twin-screw extruder having a dissolution part in which the temperature of a feed is 110 to 150 degrees Celsius and a precipitation part in which the temperature of a barrel is 0 to 60 degrees Celsius, to generate amorphous Palatinose (claim 1). A Palatinose solution is concentrated and cooled so as to form crystals, or so as to form an amorphous material. The amorphous Palatinose means the latter (page 2, lower right column, lines 4 to 7).

Patent Literature No.3 describes a method for producing crystallized powdery sorbitol, wherein a temperature of melted sorbitol which comprises a fat or oil and/or a surfactant but does not substantively comprise water is adjusted to a temperature at which said sorbitol does not solidify and crystalline sorbitol seeds do not melt and, after sorbitol seed crystals are added to the melted sorbitol and premixed or simultaneously with the addition of sorbitol, a shearing force is applied on the melted sorbitol in a certain time period at 60 to 85 degrees Celsius to crystallize sorbitol(claim 1).

Patent Literature No.4 describes a method for producing powdery or granular crystalline maltitol, characterized in that maltitol seed crystals are added to a maltitol solution having a water content of 1 to 15 wt% below the melting point of the maltitol seed crystals, and the maltitol solution is kept being kneaded to apply a shearing force on the solution in the presence or absence of an oil or fat and/or a surfactant (claim 1).

Patent Literature No.5 describes a method for producing crystalline maltitol, and syrup-comprising maltitol crystals, characterized in that the following steps are consecutively conducted: 1) a first step in which a syrup having a concentration of 30 to 75 wt% where 81 to 90 wt% of a solid content is maltose is catalytically hydrogenated to obtain a corresponding sugar alcohol syrup, 2) a second step in which the sugar alcohol syrup is fed to a tower packed with a cation exchange resin to chromatographically obtain a maltitol-rich syrup fraction in which the solid content comprises maltitol of 92 to 99.9 wt%, and 3) a third step in which, after the maltitol-rich syrup fraction is concentrated, a part of the concentrated syrup is crystallized in the presence of seed crystals to collect crystalline maltitol, and the remaining part of the concentrated syrup is spray-dried or cooled and kneaded in the presence of seed crystals to obtain syrup-comprising maltitol crystals (claim 1).

Patent Literature No.6 describes a method for producing crystalline maltitol and syrup-comprising maltitol crystals, characterized in that the following steps are consecutively conducted: 1) a first step in which a syrup where 40 to 75 wt% of a solid is maltose is catalytically hydrogenated to obtain a corresponding sugar alcohol syrup, 2) a second step in which the sugar alcohol syrup is fed to a tower packed with a cation exchange resin to chromatographically obtain a fraction rich in sorbitol, a syrup fraction (a) comprising maltitol of 80.5 to 86.5 wt% in a solid, and a fraction comprising polyols having a degree of polymerization (DP) of 3 or more, 3) a third step in which the maltitol comprising syrup is fed to a tower packed with a cation exchange resin to chromatographically obtain a sorbitol rich fraction, a syrup fraction (b) in which the solid content comprises maltitol of at least 97.5 wt %, and a fraction comprising polyols having a degree of polymerization of 3 or more, 4)a fourth step in which the maltitol-comprising syrup fraction (b) is concentrated, and crystallized to obtain crystalline maltitol and a mother liquid in which the solid content comprises maltitol of 90 wt% or more, and 5) a fifth step in which the mother liquid obtained in the fourth step is spray-dried or cooled and kneaded in the presence of seed crystals to obtain syrup-comprising maltitol crystals (claim 1).

Patent Literature No. 7 describes a method for continuously producing syrup-comprising maltitol crystals, characterized in that the following steps are continuously conducted: a step where an aqueous maltitol solution having a concentration of 92 to 98 wt% and a maltitol purity in the solid content of 88 wt% or more is continuously introduced into a vessel and stirred to generate maltitol crystals so as to produce a maltitol slurry, and a step where obtaining syrup-comprising maltitol crystals, the maltitol slurry is cooled and kneaded to form a maltitol magma and the magma is extruded through an extrusion orifice to obtain syrup-comprising maltitol crystals (claim 1).

Patent Literature No. 8 describes a method for manufacturing crystalline isomaltulose from sucrose, comprising the steps of 1) contacting an α-glucosyltransferase enzyme to aqueous sucrose solution or slurry under the condition wherein the α-glucosyltransferase enzyme is active; in which said condition is maintained after the concentration of isomaltutose in the reaction mixture reaches the point at which crystals are formed, and 2) separating the reaction mixture into crystalline isomaltutose and remaining syrup (claim 1).

Patent Literature No.9 describes a process for the enzymatic production of isomaltulose, characterized in that at least the isomaltulose-forming enzyme system of an isomaltulose-forming micro-organism is immobilized and then the immobilized enzyme system is contacted with a sucrose solution to convert at least part of the sucrose to isomaltulose (claim 1).

Patent Literature No.1: Japanese Patent Application Laid-Open No. SHO-56-117796
Patent Literature No.2: Japanese Patent Application Laid-Open No. HEI-03-240463
Patent Literature No.3: Japanese Patent Application Laid-Open No. HEI-05-294862
Patent Literature No.4: Japanese Patent Application Laid-Open No. HEI-06-7110
Patent Literature No.5: Japanese Patent Application Laid-Open No. HEI-09-19300
Patent Literature No.6: Japanese Patent Application Laid-Open No. HEI-10-17589
Patent Literature No.7: Japanese Patent Application Laid-Open No. 2004-346081
Patent Literature No.8: European Patent Application Publication EP 1 550 666 A1
Patent Literature No.9: European Patent Application Publication EP 0 028 900 A1

### SUMMARY OF THE INVENTION

### PURPOSE OF THE INVENTION

As described above, the saccharide composition of the isomaltulose-containing saccharide liquid obtained by allowing an enzyme, alpha-glucosyltransferase, to act on a sucrose liquid, is isomaltulose of 60 to 90 mass%, trehalulose of 5 to 35 mass%, glucose of 0.2 to 5 mass% and fructose of 0.2 to 5 mass%. Trehalulose is non-crystalline, i.e. liquid. Therefore, without a centrifugation step, it is difficult to obtain a solid material from the isomaltulose-containing liquid, because of a non-crystalline saccharide liquid consisting mainly of trehalulose. Accordingly, the isomaltulose-containing saccharide liquid itself could not be sold as a solidified or powderized isomaltulose product. As a conventional solidified or powderized isomaltulose product, the above-mentioned crystalline Palatinose IC (Mitsui Sugar Co., Ltd.) may be mentioned. This product is isomaltulose crystals. In order to obtain isomaltulose crystals, the crystallization step and the centrifugation step were required, as above-described. The centrifugation step is essential especially for separating isomaltulose crystals from the non-crystalline saccharide liquid. In contrast, isomaltulose accounts for about 80 mass% of the saccharides in the saccharide liquid obtained by allowing alpha-glucosyltransferase to act on a sucrose liquid. Therefore, if the saccharide liquid itself can be solidified or powderized, such a solidified or powderized product can be sold as an isomaltulose product, though its isomaltulose purity is lower than that of conventional ones. A purpose of the present invention is to provide a method for producing an isomaltulose product which can be sold as a solid product or a powdery product, without the aforesaid centrifugation step. Another purpose of the present invention is to provide a solid material comprising the aforesaid non-crystalline saccharide liquid.

### MEANS FOR ACHIEVING THE PURPOSES

The present text describes a method for producing a solid material from a saccharide liquid comprising isomaltulose, wherein said saccharide liquid is obtained by allowing an alpha-glucosyltransferase which produces isomaltulose from sucrose to act on a sucrose liquid. This method comprises the following steps: heating said saccharide liquid to adjust a solid content of said saccharide liquid to 77 to 96 mass%, subjecting a mixture resulting from the aforesaid step to a shearing force to generate crystal nuclei, while keeping a temperature of the product at 70 to 120 degrees Celsius, and cooling the mixture to obtain the solid material. The present invention provides a method for producing a solid material from a saccharide liquid comprising isomaltulose, wherein said saccharide liquid is obtained by allowing an alpha-glucosyltransferase to act on a sucrose liquid. This method comprises the following steps: heating said saccharide liquid to adjust a solid content of said saccharide liquid to 77 to 96 mass%, subjecting a mixture resulting from the aforesaid step to a shearing force to generate crystal nuclei, while keeping a temperature of the product at 70 to 120 degrees Celsius, and cooling the mixture to obtain the solid material, wherein the solid material comprises isomaltulose of an amount of 70 to 90 mass% and trehalulose of an amount of 8 to 25 mass%, and is an aggregate of isomaltulose and non-crystalline saccharide liquid which comprises trehalulose. Also, the present text describes a solid material comprising isomaltulose of an amount of 70 to 90 mass%, wherein the solid material is an aggregate of crystals and of non-crystalline saccharide liquid and wherein a median diameter of the crystals is 0.1 to 20 um, as determined by laser diffraction particle size distribution measurement. Also, the present invention provides a solid material comprising isomaltulose of an amount of 70 to 90 mass% and trehalulose of an amount of 8 to 25 mass%, wherein the solid material is an aggregate of isomaltulose crystals and of non-crystalline saccharide liquid which comprises trehalulose and wherein a median diameter of the crystals is 0.1 to 20 um, as determined by laser diffraction particle size distribution measurement.

### EFFECTS OF THE INVENTION

According to the method of the present invention, an isomaltulose-containing saccharide liquid obtained by allowing an alpha-glucosyltransferase which produces isomaltulose from sucrose to act on a sucrose liquid is solidified or powderized without the previous centrifugation step. Thus, without centrifuging the saccharide liquid, the isomaltulose-containing saccharide liquid, which comprises a non-crystalline saccharide liquid such as trehalulose, is solidified as a whole . Consequently, an isomaltulose-rich solid or powdery material is obtained.

The solid material obtained by the present method can be sold as a solid or powdery isomaltulose product.

### BRIEF DESCRIPTION OF THE DRAWINGS

Fig.1 is a schematic illustration of crystals and non-crystalline saccharide liquid in the present solid material.
Fig.2 is a copy of a microscopic photograph of the present solid material.
Fig.3 is a copy of a microscopic photograph of another present solid material.
Fig.4 is a copy of a microscopic photograph of another present solid material.
Fig.5 is a copy of a microscopic photograph of the present solid material which was crushed by hands.

### EMBODIMENTS OF THE INVENTION

In the present invention, "isomaltulose" is a di-saccharide in which glucose is bound to fructose via an alpha-1,6-glucosyl bond. Isomaltulose is called also as Palatinose (trademark), and hereinafter referred to as Palatinose.

In the present invention, "an enzyme which produces isomaltulose from sucrose" is an enzyme that generates isomaltulose from sucrose. The enzyme is, namely, an alpha-glucosyltransferase. The alpha-glucosyltransferase is derived from, for example, Protaminobacter rubrum, Serratia plymuthica, Erwinia rhapontici, or Klebsiella sp..

In the present invention, "a saccharide liquid comprising isomaltulose" is obtained by allowing an enzyme which produces isomaltulose from sucrose to act on a sucrose liquid. The sucrose liquid may be any kind of raw material for the enzyme to produce isomaltulose. For example, the sucrose liquid may be a raw liquor, a brown liquor, a carbonated liquor, and a fine liquor obtained in sugar production. The sucrose liquid may comprise sucrose in an amount of 5 to 60 mass%, especially 10 to 50 mass%, in order to optimize the enzyme reaction. The sucrose liquid may comprise saccharides other than sucrose. It is preferred that the amount of sucrose is 97 mass% or more, based on a total mass of all saccharides contained in the sucrose liquid. The enzyme may be allowed to act on the sucrose liquid according to the method described in Japanese Patent Application Laid-Open No. SHO-57-39794, but not limited to it. The saccharide liquid comprising isomaltulose is obtained by allowing the enzyme to act on the sucrose liquid. The saccharide liquid obtained by allowing the enzyme to act on the sucrose liquid (isomaltulose-containing saccharide liquid) comprises other saccharides besides isomaltulose. Such other saccharides results from the aforesaid enzyme action, such as trehalulose, fructose, glucose, sucrose, isomaltose, and isomelezitose. The isomaltulose-containing saccharide liquid may comprise minerals and/or amino acids. The saccharide liquid may comprise other components. Such other components are added, for example, to keep concentrations of components contained in the saccharide liquid constant among batches. In the method of the present invention, compositions of saccharides in the saccharide liquid are measured in a routine method in the present technical field, such as high-performance liquid chromatography.

Isomaltulose accounts for 70 to 90 mass%, preferably 72 to 89 mass%, more preferably 74 to 88 mass%, even more preferably 75 to 85 mass%, of saccharides contained in the isomaltulose-containing saccharide liquid. In calculating this isomaltulose percentage, a denominator is a total mass of isomaltulose, trehalulose, fructose, glucose, sucrose, and isomaltose contained in the isomaltulose-containing saccharide liquid. A mass of a saccharide is calculated as that of its anhydride. When the percentage of isomaltulose is too low, the saccharide liquid can not be solidified. The percentage of isomaltulose may be higher than the aforesaid upper limit, but generally the percentage is determined, depending upon an isomaltulose percentage in the saccharide liquid resulting from the enzyme action in view of production efficiency, but not exceeding the aforesaid upper limit.

The mass percentage of trehalulose, relative to the total mass of saccharides contained in the isomaltulose-containing saccharide liquid, may be, for example, 8 to 25 mass%, particularly 9 to 20 mass%, more particularly 10 to 18 mass%. The mass percentage of glucose, relative to the total mass of saccharides comprised in the isomaltulose-containing saccharide liquid, may be, for example, 0.1 to 5 mass%, particularly 0.2 to 4 mass%, more particularly 0.3 to 3 mass%. The mass percentage of fructose, relative to the total mass of saccharides contained in the isomaltulose-containing saccharide liquid, may be, for example, 0.1 to 5 mass%, particularly 0.2 to 4 mass%, more particularly 0.3 to 3 mass%. Also in calculating these percentages, a denominator is a total mass of isomaltulose, trehalulose, fructose, glucose, sucrose, and isomaltose contained in the saccharide liquid, as in calculating the mass percentage of isomaltulose. Masses of these saccharides are calculated as those of these anhydrides.

The saccharide liquid comprising isomaltulose is a solid material.

For example, saccharides other than isomaltulose may be dissolved, suspended or dispersed in the liquid, or precipitated in the liquid.

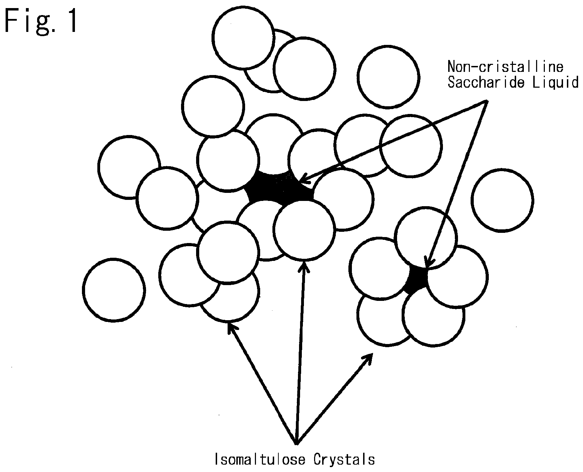

In the present invention, "a solid material" is an aggregate of crystals and non-crystalline saccharide liquid. The crystals are confirmed to be isomaltulose crystals. In the solid material of the present invention, the non-crystalline saccharide liquid is considered to be surrounded by the isomaltulose crystals. Fig.1 shows a schematic illustration of the state of the crystals and the non-crystalline saccharide liquid in the present solid material.

The crystals are confirmed to be isomaltulose crystals by a microscope, for example a digital microscope (KH-7700, HIROX Co., Ltd.). The solid material is placed in a plastic bag and pressed with fingers or a stick from the outside of the bag to obtain a crushed material. Crystals are confirmed by observing the crushed material by a microscope . Thus, the crystals seem transparent under a microscope. A solidified material of multiple components or multiple saccharides is not transparent. The crystal has an elongated form. In some cases, several crystals aggregate where crystals contact with each other at a surface. Among saccharides comprised in the saccharide liquid, saccharides other than isomaltulose are considered not to have been crystallized. For example, trehalulose is non-crystalline. Saccharides present in a low percentage, such as glucose, fructose, and sucrose, are considered to be in a syrup and not in crystals.

A median diameter of the crystals is 0.1 to 20 um, more particularly 0.2 to 15 um, even more particularly 0.3 to 10 µm. If the median diameter is too large, it takes much time for crystals to aggregate so that the production is less efficient. If the median diameter is too small, a solid material is not formed. The median diameter is measured by laser diffraction particle size distribution measurement.

The method for the measurement is known in the present technical field. Particularly, the laser diffraction particle size distribution measurement is carried out by a laser diffraction particle size distribution analyzer (SALD-2000J, SHIMADZU CORPORATION), as follows. The solid material is placed in a plastic bag, and pressed with fingers or sticks from the outside of the bag to obtain a crushed material. The crushed material is added to 2-propanol in a vial. Then, the vial is shaken several times, especially 5 to 10 times, to suspend the crushed material. After suspending the crushed material, the vial is left for several seconds, especially about 5 to 10 seconds, to obtain a supernatant of the suspension. The turbidity of the supernatant is adjusted to 2.0 or less with 2-propanol. The adjusted suspension is subjected to the laser diffraction particle size distribution measurement to obtain a median diameter.

Among commercially available isomaltulose products, crystalline Palatinose IC (Mitsui Sugar Co., Ltd.) has a median diameter of 350 to 450 um, as determined by international standard sieves (ISO) . On the other hand, in the present invention, the median diameter of the crystals is 0.1 to 20 um. Thus, the crystals having particle diameters much smaller than that of the crystalline palatinose IC are generated and the crystals aggregate in the invention.

The present solid material comprises isomaltulose and other saccharides. Mass percentages of saccharides in the present solid material are same as those in the aforesaid saccharide liquid comprising isomaltulose. The non-crystalline saccharide liquid in the present solid material comprises trehalulose, fructose, glucose, sucrose, isomaltose, and/or isomelezitose. The non-crystalline saccharide liquid may also comprise isomaltulose. Furthermore, the non-crystalline saccharide liquid may comprise components which are not saccharides and contained in the saccharide liquid obtained by allowing an enzyme which produces isomaltulose from sucrose to act on a sucrose liquid, such as minerals and/or amino acids. The non-crystalline saccharide liquid may be the aforesaid syrup. In the present solid material, the non-crystalline saccharide liquid may be liquid wholly, or a part of the liquid is liquid and the remaining part is solid. Especially, trehalulose accounts for 40 to 99 mass%, particularly 45 to 80 mass%, more particularly 50 to 70 mass%, of the mass of the non-crystalline saccharide liquid.

The present solid material may be easily crushed by a conventional crushing means in the present technical field or by human hands. As the crushing means, for example, a pin-crusher or a hammer mill may be mentioned. By crushing, powder may be obtained which consists of crystals having the aforesaid median diameter. Particles may also be obtained which has a median diameter (particle size) of, for example, 20 µm to 6 mm, especially 30 µm to 5 mm. The median diameter may be adjusted according to applications of the solid material. The median diameter may be measured by, for example, a laser diffraction particle size distribution analyzer (SALD-2000J, SHIMADZU CORPORATION).

The isomaltulose content of the present solid material may be 70 to 90 mass%, especially 72 to 89 mass%, more especially 74 to 88 mass%, even more particularly 75 to 85 mass%, even more especially more than 80 to 85 mass%. Consequently, the solid material may be used as an alternative to the aforesaid commercially available isomaltulose products. Furthermore, because of omission of the centrifugation step in the production process of the aforesaid commercially available isomaltulose products, time and energy necessary for solidifying or powderizing the liquid are saved, which leads to a highly enhanced efficiency. Also, the isomaltulose-containing saccharide liquid can be solidified as a whole, without leaving a syrup which forms in the conventional production process of isomaltulose crystals.

The water content of the present solid material as a whole may be 0.5 to 9 mass%, especially 0.55 to 8 mass%, more especially 0.8 to 7 mass%. If the water content is too high, the material will be like a fondant and will be unsolid. The present solid material may be dried so that the water content is below the aforesaid ranges. However, such a drying process is unfavorable in view of production efficiency. Among commercially available isomaltulose products, for example, the crystalline Palatinose IC (Mitsui Sugar Co., Ltd.)has a water content of 0.2 mass% or less, and powdery Palatinose ICP (Mitsui Sugar Co., Ltd.) has a water content of 0.3 mass% or less. Thus, the present solid material has a water content higher than those of commercially available isomaltulose products . The aforesaid water content is calculated, based on difference between a mass of the solid material before vacuum drying at 75 degrees Celsius for 3 hours and that after the vacuum drying (this method is hereinafter referred to as "vacuum drying method").

Alternatively, the water content may be measured by an infrared water content meter (FD-600-2, Kett Electric Laboratory, drying by heating at 65 degrees Celsius for 20 minutes) in order to shorten a time period for measurement. A water content measured by the vacuum drying method correlates with a water content measured by an infrared water content meter. A water content measured by an infrared moisture meter is converted to that measured by the vacuum drying method according to the following formulae: when the solid material are particles passing through 2 mm openings and retained on 600 µm openings, y=0. 9408x-0.3698 (R²=0.9979) ; when the solid material are particles passing through 600 µm openings, y=0.9828x-0.1879 (R²=0.997) . In these formulae, y is a water content obtained by the vacuum drying method, x is a water content measured by an infrared water content meter. R² is a determination factor in the aforesaid correlation. For example, a water content of 2.8 mass% for a solid material passing through 2mm openings and retained on 600um openings and a water content of 2.9 mass% for a solid material passing through 600um openings, as measured by an infrared moisture meter, correspond to a water content of 3 mass%, as measured by the vacuum drying method.

A color value of a liquid obtained by adding water to the present solid material so as to give a Brix of the liquid of 25 degrees and a pH of the liquid of 7 is preferably 600 or less, more preferably 500 or less, even more preferably 400 or less, 300 or less, or 200 or less, most preferably 100 or less. On account of this color value, the appearance is like that of the conventional isomaltulose product. A method for determining the color value is like a method for determining a color value of white sugar, which method is described in "Seito-Binran" (edited by the Research Society of Japan Sugar Refineries' Technologists, published on June 30, 1962, Asakura Publishing Co., Ltd.), with following modification: Brix is changed to 25 degrees, a pH is changed to 7, and a cell length is changed to 5 cm. The concrete procedures for the measurement are as follows. Water is added to the solid material so as to give a Brix of the liquid of 25 degrees, and a pH of the liquid is adjusted to 7 by a 1N hydrochloric acid solution or sodium hydroxide solution. Absorbances at wavelengths of 420nm and 720nm are measured by a spectrophotometer on the adjusted liquid. From the measured absorbances, the color value (AI) is calculated according to the following formula: AI={(-logT₄₂₀)-(-logT₇₂₀)}/(b × c)X1000, wherein -logT₄₂₀ is an absorbance at 420nm, -logT₇₂₀ is an absorbance at 720nm, b is a cell length (cm), and c (g/ml) is a mass of saccharides contained in 1ml of the adjusted liquid, calculated on the basis of the Brix of the adjusted liquid.

The present solid material comprises isomaltulose in an amount of 70 to 90 mass%, especially 72 to 89 mass%, more especially 74 to 88 mass%, more especially 75 to 85 mass%, more especially more than 80 to 85 mass%. The mass percentage of isomaltulose is a mass percentage of anhydrous isomaltulose, relative to the total mass of the solid material. The present solid material may comprise trehalulose in an amount of 8 to 25 mass%, especially 9 to 20 mass%, more especially 10 to 18 mass%. The percentage of trehalulose is a mass percentage of anhydrous trehalulose, relative to the total mass of the solid material. The present solid material may comprise glucose in an amount of 0.1 to 5 mass%, especially 0.2 to 4 mass%, more especially 0.3 to 3 mass%, relative to the total mass of the present solid material. The present solid material may comprise fructose in an amount of 0.1 to 5 mass%, especially 0.2 to 4 mass%, more especially 0.3 to 3 mass%, relative to the total mass of the present solid material.

Each step of the present method will be described below.

In the method of the present invention, a solid content of the saccharide liquid is adjusted to 77 mass%. If the solid content is too low, a solid material is not obtained in the subsequent step with a shearing force-applying device and the saccharide liquid is, for example, like a fondant. If the solid content is too high, the saccharide liquid is like a candy, so that particles, especially isomaltulose fine crystals (crystal nuclei) are not formed in the subsequent step with a shearing force-applying device. The adjustment of the solid content is preferably carried out by heating for better production efficiency, but may be carried out by any other methods. The heating step may be carried out by any routine manner known in the present technical field. For example, the saccharide liquid is put in a vessel, and is heated by a heater, while being stirred. As examples of the heater, an evaporator, a vacuum pan, an effect evaporator, and a thin-film evaporator may be mentioned. By this heating step in the present method, the temperature of the saccharide liquid may reach 108 to 122 degrees Celsius, especially 110 to 120 degrees Celsius, more especially 112 to 118 degrees Celsius, so that the aforesaid solid content is achieved. In the present invention, this heating step may be conducted at normal pressure. If the temperature in the heating step is too low, solid material is not obtained in the subsequent step with a shearing force-applying machine, and the saccharide liquid is, for example, like a fondant. If the temperature is too high, the saccharide liquid is like a candy, though the saccharide composition is not affected. In order to achieve the aforesaid solid content quicker, this heating step may be conducted at a reduced pressure . The aforesaid solid content may be attained by, for example, heating the saccharide liquid to at 72 to 86 degrees Celsius at a reduced pressure of 160 mmHg (degree of vacuum, -600 mmHg), at 64 to 77 degrees Celsius at a reduced pressure of 110 mmHg (degree of vacuum, -650 mmHg), or at 50 to 62 degrees Celsius at a reduced pressure of 60 mmHg (degree of vacuum, -700 mmHg) .

In the present invention, "a solid content" is a concentration of solid components comprised in a liquid. In the present invention, the solid content is determined in a routine method known in the present technical field. For example, several solutions having different concentrations are prepared and dried at 75 degrees Celsius and a reduced pressure for 3 hours. Then, solid contents of these solutions are obtained. Meanwhile, Brixs of these solutions are measured separately. Based on the solid contents and the Brixs of these solutions, a relationship (for example, a linear relationship) between the solid content and the Brix is obtained. A solid content of a solution is obtained, based on the relationship.

In the present method, a preparation having the adjusted solid content is subjected to a step for applying a shearing force on the preparation, while keeping the temperature of the preparation at 65 to 120 degrees Celsius to form crystal nuclei. Thus, this step is shearing treatment for generating crystal nuclei in the preparation. In the present invention, this step is conducted by a sharing force-applying device.

In the present invention, "a shearing force-applying device" is a device which applies a shearing force on a material, especially a device which kneads a highly viscous material (a device by which forces of shifting a material are applied in one direction and an opposite direction to grind, knead, or mix the material), especially a device having a function as a kneader, more especially a device which can heat and scrape a material so that the processed material is not left, adhered to a vessel of the device. The device may be a kneader, an extruder, a kneader, an agitator, and a universal mixing and agitating machine. As the kneader, for example, KRC(trademark) kneader (Kurimoto, Ltd.) may be mentioned. As the agitator, for example, Kenmix (Aicohsha Manufacturing Co.,Ltd.) may be mentioned. SC processor may also be used.

In the present method, crystal nuclei are generated in the shearing treatment by a shearing force-applying device. It is considered that the aforesaid solid material is formed due to the generation of the crystal nuclei. A preferred shearing treatment is those in which the crystal nuclei are generated. Concretely, a preferred shearing treatment is carried out by the shearing force-applying devices and the processing conditions described below. On account of the following shearing force-applying machines and processing conditions, a time for the solidification is shortened and the production is more efficient.

As a shearing force-applying device having a smaller volume, a kneader, an agitator, and a universal mixing and agitating machine may be mentioned. These devices have a bowl-shaped vessel (having a rounded bottom surface) and agitating blades for kneading with a vertical axis (such as that for a bread dough) . A feed is trapped in gaps (clearance) between the blades and the vessel and kneaded by the blades. As examples of these devices, Kenmix (Aicohsha Manufacturing Co., Ltd.) and universal mixing and agitating machine, Kanto Mixer (KANTO KONGOKI INDUSTRIAL CO., LTD.) may be mentioned. Among these machines, a small desktop-type device may have a rotation speed of 100 to 500 rpm, a device having a vessel volume of 5 kg or less may have a rotation speed of 60 to 220 rpm, and a machine having a vessel volume of 10 kg or less may have a rotation speed of 150 to 400 rpm. A peripheral velocity at the blade tip on which the strongest force is applied may be 200 to 900 cm/min, especially 300 to 800 cm/min. The velocity may be larger than this, but such a high velocity may cause the material to scatter from the vessel. In these devices, an area on which the force is applied is small. The force is applied in an obliquely downward direction, and the preparation flows upwards. Therefore, the time period required for the shearing treatment (referred to as "processing time" hereinafter) may be so long as, for example, 1 to 40 minutes, especially 2 to 30 minutes. The processing time is a time period for which a shearing force is applied on the preparation, and is for example a time period for which the preparation is kneaded or agitated.

As a shearing force-applying device having a larger volume, a horizontal axis-type device, especially a horizontal axis-type kneader, extruder, or SC processor, may be mentioned. These devices have radial blades, sigma blades, or point-symmetry (for example, oval-shaped or triangle-shaped) blades, which are fixed to an axis in a cylindrical vessel and rotate in the vessel. Therefore, a shearing force is applied on a feed from all directions, while the preparation passes through a gap (clearance) between the blades and the vessel. In a case of a kneader, kneading paddles are provided in a cylinder where the product passes through. An axis of a kneader is fixed at both ends of the axis. An inlet for a feed is placed near one end of the axis, whereas an outlet is placed near the other end of the axis. A clearance between the cylinder and the paddles in the kneader is small, so that the material is unlikely to stay in the vessel and the preparation may be scraped out of the vessel. As the kneader, for example, S2KRC(trademark) kneader (Kurimoto, Ltd.) may be mentioned. With a paddle diameter of 50 mm, a rotation speed of the kneader may be 90 to 500 rpm, preferably 100 to 360 rpm, especially 200 to 250 rpm. Preferably, the paddles are of a mixing-type. In a case of an extruder, an axis of the extruder is fixed at one end. The other end of the axis is open, which is an outlet. Usually, a screw or screws are provided in a cylinder. When an extruder is used for a shearing process, kneading disks are provided in the cylinder. The larger the paddle diameter of the kneader is or the larger the cylinder diameter of the extruder is, the greater shearing force is applied even at the same rotation speed, so that a smaller rotation speed is enough. For a horizontal axis type kneading device such as a kneader or an extruder, a clearance may be 0.3 to 5 mm, especially 0.5 to 3 mm, and a peripheral velocity may be 20 to 90 cm/min, especially 30 to 80 cm/min. In the horizontal axis type shearing force-applying device, a shearing force is always applied on the preparation from all directions and, therefore, a processing time of, for example, 2 to 60 seconds, especially 3 to 40 seconds, more especially 4 to 30 seconds is enough. The processing time is a time period for which a shearing force is applied on the preparation. The processing time is calculated according to the following formula: processing time = (an amount of the material residing in the shearing force-applying device) / (a feed rate of the material) . For example, when S2KRC kneader is used at a filling ratio of 10 volume%, a vessel volume of the cylinder is 1,200 mL and a percentage of a space not occupied by the paddles is 40 volume%, and thus the space is 480 ml. Therefore, the amount of the material in a kneader during the shearing process is 48 mL (about 50g). In such a case, if the feeding rate is 600 g/min, or 10 g/second, the processing time is 5 seconds according to the aforesaid formula. The feeding rate may also be calculated as an amount per time of the processed material discharged from the kneader. Also for devices other than S2KRC kneader, the above-described calculation is used to calculate a processing time.

A temperature of the material during the shearing process is 65 to 120 degrees Celsius. If the temperature is too low, a viscosity of the material is so high that isomaltulose nuclei do not sufficiently generate. If the temperature is too high, a degree of supersaturation is insufficient, so that crystal nuclei do not sufficiently generate. Other conditions for the process, such as a type of a device, a peripheral velocity, and a processing time may be properly adjusted so that crystals having the aforesaid median diameter are obtained in the present solid material.

In the present invention, a kneader is preferably used as the shearing force-applying machine in order to shorten a time necessary for solidification. When a kneader is used as the shearing force-applying device in the present invention, a preferred temperature of the material in the shearing process is 65 to 120 degrees Celsius. By maintaining a vessel of the kneader at the aforesaid temperature, the temperature of the material is also maintained at the same temperature.

In the method of the present invention, crystal nuclei generated by the shearing process may grow in a subsequent cooling step to become crystals in the present solid material. However, the crystal nuclei generated in the shearing process may not grow in the subsequent cooling step and may be present as such in the present solid material.

In the present invention, seeds are optionally added to the material in the shearing process for nucleation. The seeds are considered to promote generation of isomaltulose crystals in the preparation. The seeds may be such that is routinely used in the present technical field, such as crushed isomaltulose crystals, especially Palatinose ICP (Mitsui Sugar Co., Ltd.), or one obtained by simply drying the isomaltulose-comprising liquid used in the present invention at a reduced pressure (75 degrees Celsius, 2 hours) and then crushing by a hammer mill (SAMPLE-MILL KIIW-1, Fuji Paudal Co., Ltd.), or one obtained by crushing the present solid material.

After the shearing process, the processed material is cooled to obtain the present solid material. The cooling step is conducted by leaving the processed material at room temperature or, if necessary, by forced-cooling. For example, the liquid obtained from the shearing force-applying device is allowed to flow and cool on a cooling tray in a thickness of the liquid of 1 to 30 mm, preferably 2 to 20 mm, more preferably 3 to 15 mm. A steel belt may be mentioned as a cooling device. The processed material may be cooled to, for example, room temperature, or 0 to 60 degrees Celsius, more especially 10 to 50 degrees Celsius, even more especially 20 to 40 degrees Celsius.

The present solid material may be crushed into particles, for example, granules or powder. In order to obtain particles efficiently, the present solid material is preferably dried before or after crushing or simultaneously with crushing. The crushing and the drying may be carried out by any means known in the present technical field. In order to obtain particles efficiently, a water content of the present solid material may be decreased by the drying preferably to 5 to 9 mass%. Thus, the present particles preferably have the aforesaid water content. The water content is determined by the above-described vacuum drying method.

For example, the crushing step is carried out as follows. First, the present solid material is cracked into blocks having a maximum diameter of 2 to 3 cm. The resulting blocks are crushed and dried. In this way, the particles are obtained. If necessary, the dried and crushed material is further crushed.

A device for cracking the solid material may be any device as long as it can crack the solid material into blocks having maximum diameter of 2 to 3 cm, such as a pin-mill, a hammer crasher, a roll mill, a jaw crasher, or a cutter mill, but not limited to these. A device for the crushing may be any device as long as it can crush the blocks, such as a power mill, a nibbler, a flake crusher, or an impact mill, but not limited to these devices. The drying may be carried out, for example, by a fluidized bed dryer or by other dryers known to a person having ordinary skill. The crushing and drying may be carried out by one device having functions of crushing and drying, such as Drymeister. Drymeister may have a classifying function, which will be described below.

The particles of the present invention may be classified, when necessary. Classification may be carried out by any means known to a person having ordinary skill. A sieve opening for the classification is properly determined by a person having ordinary skill. By the classification, particles are obtained 1 mass% or less of which in the particle size distribution has a particle diameter equal to or more than the aforesaid sieve opening, such as a particle diameter of 5 mm or more, 4 mm or more, 3 mm or more, 2 mm or more, 1mm or more, or 600 µm or more. A sieve having a sieve opining of 3.35 mm, 2mm, 1mm, or 600 µm according to ISO3310-1:2000 may be used. Two sieves having different sieve openings may be used. Then, particles are obtained, 1 mass% or less of which do not have a diameter between these two sieve openings, thus particles is obtained, more than 99 mass% of which have a diameter between these two sieve openings. Particles having a diameter of 1 mm or less, especially 600 µm or less, are powdery. Particles have a diameter of more than 1mm to 5 mm are granular. In the present invention, particles having a diameter of N mm or more mean that they do not pass through a sieve having a sieve opening of N mm.

The present text describes a solid material comprising isomaltulose in an amount of 70 to 90 mass%, wherein the solid material is an aggregate of crystals and non-crystalline saccharide liquid and wherein a median diameter of the crystals is 0.1 to 20 µm, as determined by laser diffraction particle size distribution measurement. The present invention provides a solid material comprising isomaltulose in an amount of 70 to 90 mass%, wherein the solid material is an aggregate of isomaltulose crystals and non-crystalline saccharide liquid which comprises trehalulose and wherein a median diameter of the crystals is 0.1 to 20 µm, as determined by laser diffraction particle size distribution measurement. The solid material is obtained by the method of the present invention. Characteristics of the solid material are as mentioned in relation with the method of the present invention.

The present invention will be further explained with reference to the Examples 2 below.

In the following Examples, a solid content was determined as follows. Several solutions having different concentrations were prepared and dried at 75 degrees Celsius and a reduced pressure for 3 hours. Then, solid contents of these solutions were determined. This drying process was conducted in a method routinely used for determining a solid content using a vacuum pump. Meanwhile, Brixes of these solutions were measured separately. Brixs were determined by a digital refractometer (RX-5000, ATAGO CO.,LTD.). Then, a linear relationship between the solid contents and Brixs of these solutions was obtained. Based on this linear relationship, the solid contents of the solutions were obtained in the following Examples.

### Example 1

### Production of a Solid Material

### 1. Heating of a Model Liquid

A model liquid for a saccharide liquid obtained by allowing an enzyme which produces isomaltulose from sucrose to act on a sucrose liquid was prepared by mixing Palatinose IC (Mitsui Sugar Co., Ltd.), Palatinose Syrup ISK (Mitsui Sugar Co., Ltd.), and water in a mass proportion: IC:ISK:water=65:24:11. Generally, an enzyme reaction liquid for producing isomaltulose is demineralized and then subjected to a step of separating Palatinose crystals. This separated Palatinose crystals are Palatinose IC (Mitsui Sugar Co., Ltd.), and syrup separated from the reaction liquid is Palatinose Syrup ISK (Mitsui Sugar Co., Ltd.) . Therefore, a mixture of Palatinose IC (Mitsui Sugar Co., Ltd.) and Palatinose Syrup ISK (Mitsui Sugar Co., Ltd.) in the aforesaid proportion can be used as a model liquid for a saccharide liquid obtained by allowing an enzyme which produces isomaltulose from sucrose to act on a sucrose liquid.

The model liquid was condensed in a heating condensor (15L volume) at normal pressure until the temperature of the model liquid reached 115 degrees Celsius . A Brix of the liquid after this condensation was about 85 degrees. A solid content of the liquid after this condensation was 89%. In this way, a preparation having a solid content of 89% was obtained.

### 2. Shearing the Preparation

The preparation was processed by S2KRC(trademark) kneader (Kurimoto, Ltd.) at a rotation speed of 228 rpm and the cylinder temperature and the feeding rate as shown in Table 1 below. As seen in Table 1, the processing time in the kneader (residence times in the kneader) was 5 to 18 seconds and the temperature of the preparation during the shearing process was 73 to 91 degrees Celsius. These processing times were calculated using the above-described calculation method for a filling ratio in the S2KRC kneader of 10%. The processed material leaving the kneader was received on a stainless steel tray so that a thickness of the processed material was 3 mm and, then, left to cool down to become solid. The time periods from the discharge to the time when the processed material solidified were recorded.

### Table 1

**Table 1: Results of the Solidification**

| | Feeding Rate (g/min) | Cylinder Temp. **(°C)** | Residence Time in the Device (s) | Material Temp. during Process **(°C)** | Result | Time for Solidification (min) |
|---|---|---|---|---|---|---|
| Example **1 - 1** | **170** | **80** | **18** | **73** | ⊚ | **10** |
| Example **1 - 2** | **170** | **90** | **18** | **83** | ⊚ | **5** |
| Example **1 - 3** | **170** | **100** | **18** | **91** | ⊚ | **13** |
| Example **1 - 4** | **340** | **90** | **9** | **86** | ⊚ | **5.5** |
| Example **1 - 5** | **500** | **90** | **6** | **88** | ⊚ | **10** |
| Example **1 - 6** | **500** | **80** | **6** | **83** | ⊚ | **6** |
| Example **1** - **7** | **600** | **75** | **5** | **82** | ⊚ | **6.5** |

In Table 1, a double circle indicates that a solid material was obtained. As seen in Table 1, the solid materials were obtained with all of the processing times and material temperatures during the process . When the material temperatures were as in Examples 1-2, 1-4, 1-6, and 1-7, the time for solidification was especially short. All of the resulting solid materials were crushed by a crusher (pin crasher) and, then, dried in fluidized bed, so that particles were obtained.

### Analyses of the Solid Materials

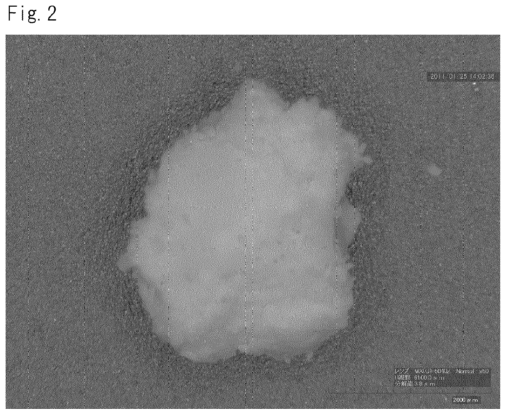

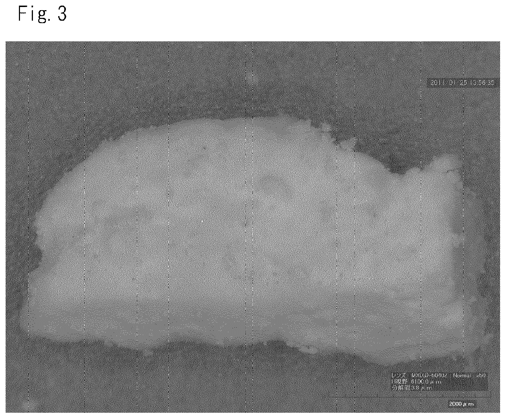

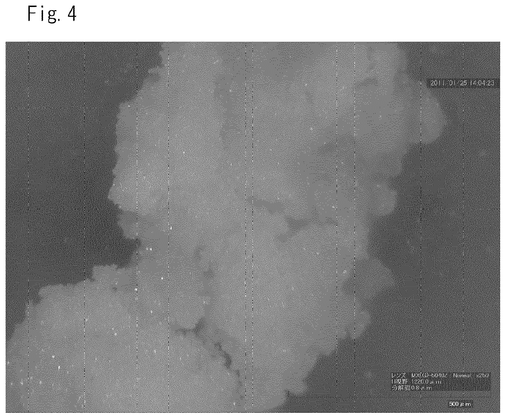

The solid material from Example 1-4 was observed under a digital microscope (KH-7700, HIROX Co., Ltd.). As seen in Figs. 2 and 3 (magnification, 50), the solid material is an aggregate of particles, especially aggregated crystals. As seen in Fig.4(magnification, 250), a size of the crystals is less than 50 µm. Next, the solid material was crushed by hands to obtain a crushed material. The crushed material was added to 2-propanol in a vial and, then, the vial was shaken 5 to 10 times to suspend the crushed material. The suspension was left for about 5 to 10 seconds to obtain a supernatant. A turbidity of the supernatant was adjusted to 2.0 or less. After adjusting the turbidity, a median diameter of crystals in the supernatant was measured three times by a laser diffraction particle size distribution analyzer (SALD-2000J, SHIMADZU CORPORATION). The results are as shown in Table 2.

### Table 2

**Table 2: Measurements of the Particle Size of Crystals**

| | Median Diameter | Mode Diameter | Mean | **25%D** | **50%D** | **75%D** |
|---|---|---|---|---|---|---|
| 1st time | **4.268** | **5.067** | **4.341** | **2.492** | **4.248** | **7.260** |
| 2nd time | **4.034** | **4.144** | **4.066** | **2.530** | **4.034** | **6.504** |
| 3rd time | **4.280** | **4.144** | **4.193** | **2.714** | **4.280** | **6.892** |
| Average | **4.194** | **4.452** | **4.200** | **2.579** | **4.187** | **6.885** |

As seen in Table 2, the average median diameter of the crystals was 4.194 µm.

A water content of the solid material of Example 1-4 was measured as follows : about 5 g of the solid material was precisely weighed on a weighing tray of about 30 g and, then, dried at 75 degrees Celsius for 3 hours in vacuum. A water content was calculated as a difference between a mass of the solid material before the vacuum drying and that after the vacuum drying. This vacuum drying was carried out in a manner routinely used in measuring a water content of a food, and using a vacuum pump. The measurement was conducted three times. The results of these three measurements are shown in Table 3.

### Table 3

**Table 3: Water Content of the Sol id Material**

| | Weight of the Weighing Tray (g) | Weight of the Solid Material (g) | Total weight of the Tray + the Solid Material after Dryied (g) | Water Content (%) |
|---|---|---|---|---|
| 1st time | **30.7524** | **5.0601** | **35.2617** | **1.5380** |
| 2nd time | **30.8266** | **5.0221** | **35.3095** | **1.5040** |
| 3rd time | **30.5963** | **5.0541** | **35.1015** | **1.5400** |
| | | | Average | **1.53** |

As seen in Table 3, the average water content of the solid material was 1.53 mass%.

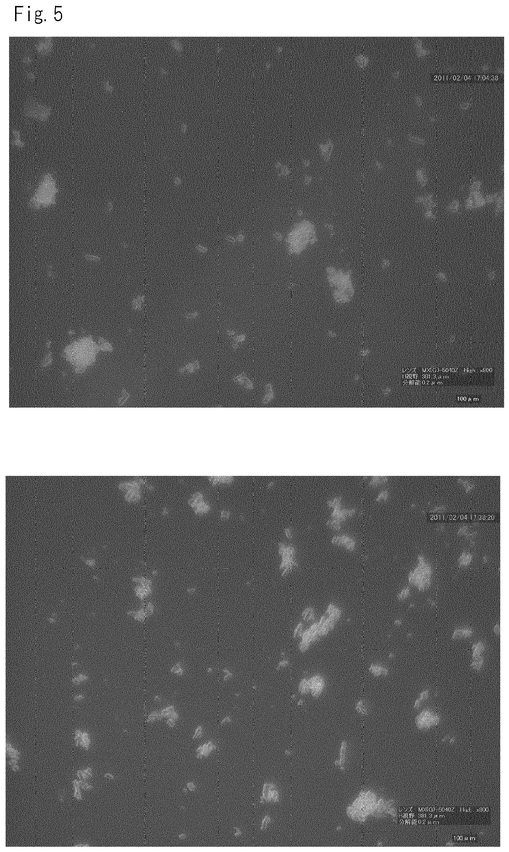

Each of the solid material of Example 1-4 was crushed by hands and, then, observed under a digital microscope (KH-7700, HIROX Co., Ltd.). An image in the observation is shown in Fig.5. As seen in Fig.5, the particles constituting the solid material are transparent. Therefore, these particles are isomaltulose crystals.

### Example 2

### Production of a Solid Material

### 1. Heating of a Model Liquid

A model liquid for a saccharide liquid obtained by allowing an enzyme which produces isomaltulose from sucrose to act on a sucrose liquid was prepared by mixing Palatinose IC (Mitsui Sugar Co., Ltd.), Palatinose Syrup ISK (Mitsui Sugar Co., Ltd.), and water in a mass proportion: IC:ISK:water=58.3:21.7:20. The composition of the model liquid is shown in Table 4 below.

### Table 4

**Table 4: Saccharide Composition of the Model Liquid (wt%)**

| | |
|---|---|
| Fructose | **2.4** |
| Glucose | **2.1** |
| Sucrose | **0.3** |
| Isomaltulose | **83.8** |
| Trehalulose | **10.7** |
| Isomaltose | **0.9** |
| Total | **100** |

The model liquid was put in a pan, and heated to 115 degrees Celsius or 120 degrees Celsius by an IH heater while stirring with a wooden spatula. A solid content of the model liquid was 88.5 mass% at 115 degrees Celsius and 92.3 mass% at 120 degrees Celsius. In this way, the preparations with the controlled solid content were obtained.

### 2. Shearing of the Preparation

When a temperature of the product reached 115 degrees Celsius or 120 degrees Celsius, seeds were added to the preparation and mixed by an agitator (Kenmix, Aicohsha Manufacturing Co., Ltd.) at an agitation speed level of 3. The seeds were Palatinose ICP or those obtained by merely drying the model liquid at 75 degrees Celsius and reduced pressure for 3 hours and crushing the dried preparation by a hammer mill (SAMPLE-MILL KIIW-1, Fuji Paudal Co., Ltd.) (hereinafter, referred to as "Model Liquid Seeds"). The amounts of the seeds were as described in Table 5 below. An aluminum-made beater appended to the agitator was used in the mixture. During agitation, the preparation was maintained at 70 degrees Celsius by a heater. A time period from the start of the agitation to the time when a solid material was obtained was recorded.

### Comparative Example 1

The same solidification procedures as in Example 2 were repeated except that the model liquid was heated to 125 degrees Celsius. A solid content of the model liquid at 125 degrees Celsius was 95.6 mass%.

The results of solidification were as shown in Table 5 below.

### Table 5

**Table 5: Results of Solidification**

| | Amount of the Model Liquid | Temp. in the Heating | Seeds | | | Result | Time Period of the Agitation to Solidification |
|---|---|---|---|---|---|---|---|
| | | | **ICP** | Model Liquid Seeds | Percentage | | |
| | **(g)** | **(°C)** | **(g)** | | **(%)** | | (min) |
| Example **2-1** | **500** | **115** | **4** | - | **1** | ⊚ | **20** |
| Example **2-2** | **500** | **115** | - | **107.6** | **23** | ⊚ | **5** |
| Example **2-3** | **500** | **115** | - | **179.4** | **33** | ⊚ | **4** |
| Example **2-4** | **500** | **115** | - | **287.4** | **44** | ⊚ | **3** |
| Example **2**-**5** | **1000** | **115** | **8** | **-** | **1** | ⊚ | **12** |
| Example **2-6** | **1500** | **120** | **-** | **107.6** | **9** | ○ | - |
| Comparative Example 1 | **500** | **125** | **4** | - | **1** | × | **-** |

In Table 5, a double circle indicates that a solid material was obtained; a circle indicates that a part of the material became a candy, though a solid material was obtained, and an X indicates that no solid material was obtained and a candy was formed. As seen in Table 5, when the temperature in the heating was 115 or 120 degrees Celsius, a solid material was obtained. When the temperature in the heating process was 125 degrees Celsius, the reaction liquid became a candy, and no solid material was obtained. In the both cases of the heating temperature of 115 or 120 degrees Celsius, a solid material was obtained with either seeds. Especially, when the heating temperature was 115 degrees Celsius, a solid material was obtained without a candy part.

### Reference Example: Analysis on How Much Isomaltulose Decomposed in the Heating Step

The model liquid was heated and, then, it was examined how much isomaltulose decomposed. The model liquid was heated to the temperatures indicated in Table 6 below and, then, saccharide composition was analyzed by HPLC. The results were as shown in Table 6 below.

### Table 6

**Table 6: Saccharide Composition in Var ious Temperatures of the Heat ing (wt%)**

| Temp. **(°C)** | **110** | **115** | **120** | **125** | **130** | **135** |
|---|---|---|---|---|---|---|
| Fructose | **2.4** | **2.4** | **2.4** | **2.4** | **2.4** | **2.4** |
| Glucose | **2.1** | **2.2** | **2.1** | **2.1** | **2.1** | **2.2** |
| Sucrose | **0.3** | **0.4** | **0.3** | **0.3** | **0.2** | **0.2** |
| Isomaltulose | **83.8** | **83.5** | **83.6** | **83.4** | **83.4** | **83.4** |
| Trehalulose | **10.7** | **10.6** | **10.8** | **10.9** | **10.9** | **11.0** |
| Isomaltose | **0.9** | **1.0** | **0.9** | **0.9** | **0.9** | **0.8** |
| Total | **100.0** | **100.0** | **100.0** | **100.0** | **100.0** | **100.0** |

As seen in Table 6, no great difference in the saccharide composition is found among these temperatures. Thus, isomaltulose did not decompose during the heating step.

### Example 3

A sucrose solution having 40 mass% was allowed to react with an alpha-glucosyltransferase produced by Protaminobacter rubrum CBS574.77 immobilized in a bioreactor, to obtain a reaction liquid. The obtained reaction liquid had a Brix of 41.1 degrees. Saccharide composition of the reaction liquid was isomaltulose of 83.1 mass%, trehalulose of 11.1 mass%, fructose of 1.9 mass%, glucose of 1.5 mass%, and other saccharides of 2.4 mass% . The reaction liquid was demineralized by a cation exchange resin and an anion exchange resin to obtain a demineralized liquid.

The demineralized liquid was fed to a horizontal centrifugal thin-film evaporator (Rototherm RT-2, type A, Mitsubishi Materials Techno Corporation) at a feeding rate of 43.14 kg/h to be concentrated. The concentrated liquid had a Brix of 89.8 degrees and a solid content of 93.7 mass%. The temperature of the liquid discharged from the evaporator was 112 degrees Celsius, a discharging rate was 19.86 kg/h, an evaporating rate was 23.28 kg/h, and a condensation factor was 2.17 times. The discharged liquid was subjected to the shearing treatment and the cooling step in the same conditions as in Example 1-4 with S2KRC kneader (Kurimoto, Ltd.). The temperature of the material during the shearing treatment was 88 degrees Celsius. As a result of these treatments, a solid material was obtained. The time period for solidification was 5.5 minutes. A median diameter of crystals constituting the solid material was 4.30 µm. A water content of the solid material was 1.59 mass%, as determined by the vacuum drying method.

### Example 4

A reaction liquid was obtained in the same way as in Example 3. This reaction liquid had a Brix of 40.9 degrees. Saccharide composition of the reaction liquid was isomaltulose of 81.0 mass %, trehalulose of 11.3 mass%, fructose of 2.3 mass%, glucose of 2.2 mass%, and other saccharides of 3.1 mass%. The reaction liquid was demineralized and concentrated in the same way as in Example 3 to obtain a concentrated liquid. The concentrated liquid had a Brix of 86 degrees and a solid content of 89.8 mass%. The concentrated liquid was subjected to a shearing treatment by S4KRC kneader (Kurimoto, Ltd.) with a rotation speed of 240 rpm and a feeding rate of 21 g/sec. The temperature of the concentrated liquid at the time of feeding the liquid to the kneader was 96 degrees Celsius. A jacket temperature of the kneader was 70 degrees Celsius . The processing time for the concentrated liquid (residence time in the kneader) was 12.4 seconds. The processing time was obtained according to the above-described processing time calculation method for the S2KRC kneader, but here the cylinder volume was changed to 6300 mL and a feeding rate was changed to 21 g/sec. A temperature of the concentrated liquid at the time of being discharged from the kneader after the shearing treatment was 71 degrees Celsius. After the shearing treatment, the concentrated liquid was poured on a stainless steel tray and left to cool, so that the concentrated liquid solidified to give a solid material. A time necessary for the solidification was about 6 minutes.

This solid material was roughly broken by a pin-mill(RB1212, Kurimoto, Ltd.) to obtain a roughly broken material. The broken material was blocks having a maximum diameter of 2 to 3 cm. A bulk density of the broken material in loose packing was 0.75 kg/L, as determined by ABD Powder Tester (TSUTSUI SCIENTIFIC INSTRUMENTS CO., LTD.). A water content of the broken material was 8.6 mass%, as determined by an infrared water content meter (FD-600-2, Kett Electric Laboratory) after drying by heating at 65 degrees Celsius for 20 minutes. This broken material was crushed by a crushing granulator (Powermill P-3S, Dalton Co., Ltd.), at a rotation speed of 4,000 rpm, a screen opening of 7mm, with a bottom plate to obtain a crushed material. A processing capacity of the granulator was 782 kg/h. This processing capacity was calculated, based on a mass of the crushed material leaving the granulator in 30 seconds. 45.5 mass% of the crushed material passed through a sieve having a sieve opening of 2mm according to ISO3310-1:2000. A bulk density of the crushed material was 0.59 kg/L. This crushed material was dried with a fluidized bed dryer (Midget Dryer MDB-400, Dalton Co., Ltd.) with a hot air temperature of 60 degrees Celsius and an air flow of 4.4 m³/min until an exhaust gas temperature reached 53.3 degrees Celsius. A bulk density of the dried material was 0. 62kg/L. A water content of the dried product was 2.8 mass%, as determined by the vacuum drying method. The dried material was again crushed by the aforesaid crushing granulator at a rotation speed of 4000 rpm, a screen opening diameter of 3mm, with a bottom plate to obtain particles. 78.5 mass% of the particles passed through a sieve having a sieve opining of 2 mm according to the aforesaid international standards. The particles were classified with a vibrating screening machine equipped with two sieves having openings of 2 mm or 600 µm according to the aforesaid international standards. Among the particles resulting from the classification, those passing through a 600µm sieve are referred to as "Particles of Example 4-1", and those passing through a 2mm sieve but retained on a 600um sieve are referred to as "Particles of Example 4-2". These preparation procedures were repeated thrice. Lot 1, Lot 2, and Lot 3 were obtained each for Particles of Example 4-1 and Particles of Example 4-2.

### Example 5

A solid material obtained as in Example 4 was crushed, dried, and classified by a flash dryer equipped with a crushing pin-hammer (Drymeister DMR-1, HOSOKAWA MICRON CORPORATION) at a rotation speed of a distributing rotor of 5500 rpm, a rotation speed of a classifying rotor of 170 rpm, an intake hot-air temperature of 80 degrees Celsius and an exhaust gas temperature of 72 degrees Celsius to obtain particles. A processing capacity of the flash dryer was 4.51kg/h. A water content of the particles was 2.67 mass%, as determined by the vacuum drying method. Particle sizes ranged 0.020um to 2,000 µm and a median diameter of the particles was 514.5 um, as measured by a laser diffraction particle size distribution analyzer (Mastersizer2000, Malvern Instruments Ltd.).

### Test Example 1: Characterization of the Particles Obtained in Example 4

A dissolution rate, a water content, a water activity, and a color value was determined on the following four samples: the particles of Example 4-1, the particles of Example 4-2, Powdery Palatinose ICP, Mitsui Sugar Co., Ltd. (hereinafter referred to as "ICP"), and crystalline Palatinose (Palatinose IC, Mitsui Sugar Co., Ltd., hereinafter referred to as "IC") . Methods for determining these values will be described below.

### Dissolution Rate

80g of distilled water was put in a 200ml-volume beaker and stirred with a stirrer at 300 rpm at 20 degrees Celsius in a water bath. Each 20g of the aforesaid samples was added to the water and a time period necessary for the sample to dissolve was determined. The time when the solid material became invisible in the liquid was defined as the time of dissolution. A dissolution rate was measured three times only for Lot 1 of the Particles of Examples 4-1 and 4-2.

### Water Content

A water content was determined by the aforesaid vacuum drying method.

### Water Activity

A water activity was determined by a water activity determination instrument (Novasina IC-500 AW-LAB, NIHON SIBER HEGNER, Ltd.) and a temperature/humidity sensor (Novasina enBSK, NIHON SIBER HEGNER, Ltd.) .

### Color Value

A color value was determined by the above-described method.

The results are shown in Table 7 below.

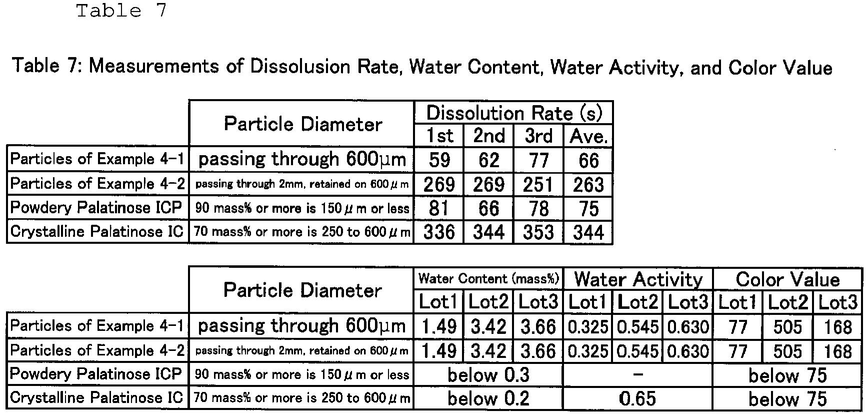

As seen in Table 7, the dissolution rate of the Particles of Example 4-1 was shorter than those of IC and ICP. Thus, a dissolution rate for the Particles of Example 4-1 was faster than those of IC and ICP. The dissolution rate for the particles of Example 4-2 was faster than that of IC.

The water contents of the Particles of Examples 4-1 and 4-2 were higher than that of IC. The water activities of the three Lots of the Particles of Examples 4-1 and 4-2 were almost the same as that of IC or lower than that of IC.

The color values of the Particles of Examples 4-1 and 4-2 were almost the same as those of ICP and IC or higher than those of ICP and IC. On account of this color value, the appearances of the Particles of Examples 4-1 and 4-2 are like that of the conventional isomaltulose product.

### Test Example 2: Production of Hard Candies and Evaluation

7 parts by mass of the Particles of Example 4-2 and 3 parts by mass of water were placed in a pan, and mixed and heated on a fire. When a liquid temperature of the mixture reached 160 degrees Celsius, the pan was removed from a fire. The liquid was poured into molds and solidified to obtain hard candies.

The same procedures as described above were repeated, except that Palatinose (crystalline Palatinose IC, Mitsui Sugar Co., Ltd.) was used instead of the Particles of Example 4-2.

The hard candies prepared from the Particles of Example 4-2 and from Palatinose IC were as transparent. When hard candies are prepared from sucrose only, crystals appears in a condensing or solidifying step, so that transparent hard candies are not obtained. It was shown that, when candies are prepared from Palatinose IC or the present solid material, transparent candies could be obtained.

### Test Example 3: Production of Yoghurt Drinks and Evaluation

### Production of Yoghurt Drinks

Yoghurt drinks were produced using the ingredients in the compositions of Tests 1 to 4 indicated in Table 8 below. The amounts of the Particles of Examples 4-1 and 4-2 and Palatinose was 1/0.45 times as much as that of sucrose in order to provide the same degree of sweetness among them. Production procedures are as follows. (1) Powdered skim milk was added to yoghurt and mixed so that lumps are not formed. (2) Milk was added to the mixture obtained in (1) and mixed, and each saccharide was added and mixed. (3) A citric acid solution of 30 mass% was added to the mixture obtained in (2) to adjust a pH at 4.5 to obtain yoghurt drinks.

### Table 8

**Table 8: Ingredient and Composition for Yoghurt Drink**

| Composition (g) | Test 1 | Test 2 | Test 3 | Test 4 |
|---|---|---|---|---|
| Yoghurt | **100** | **100** | **100** | **100** |
| Milk | **100** | **100** | **100** | **100** |
| Particles of Example 4-1 (Particle Diameter: below 0.6mm) | **31** | **-** | **-** | **-** |
| Particles of Example 4-2 (Particle Diameter: 2.0 to 0.6mm) | - | **31** | - | - |
| Sugar (granulated sugar GN, Mitsui Sugar Co., Ltd.) | - | - | **15** | - |
| Palatinose (crystal line PalatinoseIC, Mitsui SugarCo ., Ltd.) | - | - | - | **31** |
| Powdered Skim Milk | **1.5** | **1.51** | **1.5** | **1.51** |

| | | | | |
|---|---|---|---|---|
| A 30% citric acid solution was added to attain a pH of 4.5. | | | | |

### Evaluation of the Appearance

The color of the yoghurt drinks of Tests 1 to 4 were analyzed by a chromameter (CR-400, KONICA MINOLTA, INC.). The values in Table 9 are in accordance with a CIE color-difference formula L*a*b* defined by the International Commission on Illumination.

### Table 9

**Table 9: Color Differences**

| | **L∗value** | **a∗value** | **b∗value** |
|---|---|---|---|
| **Test 1** | **84.21** | **-3.65** | **5.81** |
| **Test 2** | **83.91** | **-3.67** | **5.80** |
| **Test 3** | **84.73** | **-3.51** | **5.86** |
| **Test 4** | **84.60** | **-3.65** | **5.77** |

As seen in Table 9, almost no difference was found among the yoghurt drinks of Tests 1 to 4. Thus, using the Particles of Examples 4-1 and 4-2, yoghurt drinks are produced which have the same appearance as those produced with sugar or Palatinose. Viscosities of the yoghurt drinks of Tests 1, 2, and 4 were higher than that of Test 3. This is because the amounts of saccharide in Tests 1, 2, and 4 were larger than that in Test 3.

### Evaluation of Taste

As described above, the degrees of sweetness were made equal among Tests 1 to 4. However, the yoghurt drink of Test 3 was sweetest, and the yoghurt drink of Test 4 was least sweet. Sweetnesses of Tests 1 and 2 were weaker than that of Test 3, but could be sufficiently felt.

The yoghurt drinks of Tests 1 and 2 were sourest, while that of Test 3 was least sour.

Comparing the taste of the yoghurt drink of Test 4 with those of Tests 1 and 2, the taste of Test 4 was simple and refreshing, whereas the yoghurts of Tests 1 and 2 had rich sweetness and a rich taste. No difference in taste was felt between the yoghurts of Tests 1 and 2.

### Test Example 4: Production of Whipped Creams and Evaluation

### Production of Whipped Creams

Whipped creams were produced, using the ingredients in the compositions of Tests 1 to 4 indicated in Table 10 below. The amounts of the Particles of Examples 4-1 and 4-2 and Palatinose were 1/0.45 times as much as that of sucrose in order to provide the same degree of sweetness among them. Each saccharide was added to fresh cream and whipped by a hand mixer. The whipping was stopped at the time when the cream reached stiff-peak.

### Table 10

**Table 10: Ingredient and Composition of Whipped Cream**

| Composition (g) | Test 1 | Test 2 | Test 3 | Test 4 |
|---|---|---|---|---|
| Fresh Cream | **100** | **100** | **100** | 1**00** |
| Particles of Example 4-1 (Particle Diameter: below 0. 6mm) | **16.67** | - | - | - |
| Particles of Example 4-2 (Particle Diameter: 0.2 to 0.6mm) | - | **16.67** | - | - |
| Sugar (powdery sugar, Mitsui Sugar Co., Ltd.) | - | - | **7.5** | - |
| Palatinose (powdery Palatinose ICP, Mitsui Sugar Co., Ltd.) | - | - | - | **16.67** |

No difference in texture and taste was recognized among the whipped creams of Tests 1 to 4. The whipped creams of Tests 1, 2 and 4 became foamy earlier than that of Test 3.

### Test Example 5: Production of Chocolates and Evaluation

### Production of Chocolates

Chocolates were produced, using the ingredients in the compositions of Tests 1 to 3 indicated in Table 11 below. In Tests 1 and 2, a half of the amount of the sweetener was sugar to supplement sweetness. Chocolates were produced as follows. First, the saccharide was crushed by a crusher and added to black chocolate and heated in a bowl in hot water. After the black chocolate melted, they were well mixed at 40 to 45 degrees Celsius for 5 minutes so that air was not entrapped in the chocolate. After the saccharide was mixed uniformly, the mixture was heated to 56 degrees Celsius and, then, cooled to 28 degrees Celsius by placing the bowl in cold water. Then, the mixture was heated to 31 degrees Celsius by placing the bowl in hot water, poured into molds, and cooled to obtain chocolates.

### Table 11

**Table 11: Ingredients and Compositions of Chocolates**

| Composition (g) | Test 1 | Test 2 | Test 3 |
|---|---|---|---|
| Black Chocolate (Cacao 86%) | **40** | **40** | **40** |
| Sugar (granulated sugarGN, Mitsui Sugar Co., Ltd.) | **4.8** | **4.8** | **9.6** |
| Particles of Examp l e 4-1 | **4.8** | **-** | **-** |
| Palatinose (crystalline Palatinose IC, Mitsui Sugar Co., Ltd.) | - | **4.8** | - |

### Evaluation of Taste

Compared to Test 2, the chocolate of Test 1 had richer sweetness and better smell and flavor. The chocolate of Test 3 was the sweetest, and that of Test 2 was least sweet.

The chocolate of Test 3 was bitterer than those of Tests 1 and 2. Because the chocolate of Test 2 was less sweet, only bitterness was strongly felt. The chocolate of Test 1 was very sweet and bitter, and the flavor of cacao was felt well.

### Test Example 6: Production of Ground-green-tea Sponge Cakes and Evaluation

### Production of Sponge Cakes

Sponge cakes were produced, using the ingredients in the compositions of Tests 1 to 3 indicated in Table 12 below. In Tests 1 and 2, a half of the amount of the sweetener was sugar to supplement sweetness. Sponge cakes were produced as follows. First, cake flour and ground green tea were mixed and sieved. Butter was melted. Saccharide was added to whole eggs and beaten for 12 minutes by a hand mixer, while keeping the temperature at 30 degrees Celsius by warming it in a bowl in hot water. To the beaten and foamed liquid egg, the aforesaid mixture of low-gluten flour and ground green tea was added in three portions and mixed gently. Then, the melted butter was added, and gently mixed, but not kneaded, to obtain a sponge cake dough. The dough was poured on cooking paper in a tray, and baked at 200 degrees Celsius for 15 minutes to obtain a ground-green-tea sponge cake.

### Table 12

**Table 12: Ingredients and Compositions of Ground Green Tea Sponge Cakes**

| Composition (g) | Test 1 | Test 2 | Test 3 |
|---|---|---|---|
| Whole Egg | **150** | **150** | **150** |
| Sugar (granulated sugar GN, Mitsui Sugar Co., Ltd.) | **45** | **45** | **90** |
| Particles of Example 4-1 | **45** | **-** | - |
| Palatinose (crystalline Palatinose IC, Mitsui Sugar Co., Ltd.) | **-** | **45** | **-** |
| Low Gluten Flour | **90** | **90** | **90** |
| Ground Green Tea | **10** | **10** | **10** |
| Salt-Free Butter | **15** | **15** | **15** |

Among the produced ground-green-tea sponge cakes, the sponge of Test 1 had mild sweetness and a light taste.

### Test Example 7: Production of Baton de Chocolat (bar-shaped chocolates) and Evaluation

### Production of bar-shaped chocolates

Bar-shaped chocolates were produced, using the ingredients in the compositions of Tests 1 to 3 indicated in Table 13 below. In Tests 1 and 2, a half of the amount of the sweetener was sugar to supplement sweetness. Bar-shaped chocolates were produced as follows. First, butter was kneaded to become creamy, to which the saccharide was added and mixed. Whole eggs were gradually added to the mixture. Then, sieved powder of a mixture of flour, almond powder, and cocoa powder was added to obtain a dough. The dough was squeezed from a pastry bag onto a tray, baked at 170 degrees Celsius for 15 minutes to obtain bar-shaped chocolates.

### Table 13

**Table 13: Ingredients and Compositions of Baton de Chocolat**

| Composition (g) | Test 1 | Test 2 | Test 3 |
|---|---|---|---|
| Low Gluten Four | **120** | **120** | **120** |
| Salt-Free Butter | **120** | **120** | **120** |
| Sugar (granulated sugarGN, Mitsui Sugar co., Ltd.) | **40** | **40** | **80** |
| Particles of Example 4-1 | **40** | **-** | - |
| Palatinose (crystalline Palatinose IC, Mitsui Sugar Co., Ltd.) | - | **40** | - |
| Whole Egg | **50** | **50** | **50** |
| Almond Powder | **30** | **30** | **30** |
| Cocoa Powder | **20** | **20** | **20** |
| Water | **13.3** | **13.3** | **13.3** |

The chocolate of Test 1 had less light taste than that of Test 2, but had lighter taste than that of Test 3. The chocolate of Test 1 was the bitterest.

### Test Example 8: Production of Carrot Jellies and Evaluation

### Production of Carrot Jellies

Carrot Jellies were produced, using the ingredients in the compositions of Tests 1 to 3 indicated in Table 14 below. Carrot Jellies were produced as follows. First, carrots were cut into an appropriate size and, then, processed in a mixer together with water and a lemon juice (15 seconds, 3 times) to obtain a carrot juice. Separately, a gelling agent and the saccharide was well mixed, and added to the carrot juice in a pan, well mixed, and heated to boil for 3 minutes. The boiled liquid was poured into a mold, and cooled to obtain a carrot jelly.

### Table 14

**Table 14: Ingredients and Compositions of Carrot Jellies**

| Composition (g) | Test 1 | Test 2 | Test 3 |
|---|---|---|---|
| Carrot | **100** | **100** | **100** |
| Lemon Juice | **15** | **15** | **15** |
| Water | **250** | **250** | **250** |
| Particles of Example 4-2 | **60** | **-** | - |
| Palatinose (crystal line Palatinose IC, Mitsui Sugar Co., Ltd.) | - | **60** | - |
| Sugar (granulated SugarGN, Mitsui Sugar Co., Ltd.) | - | **-** | **60** |
| Gelling Agent (WP-501, Mitsui Sugar Co., Ltd.) | | | **4** |
| Water | **13.3** | **13.3** | **13.3** |

The jellies of Tests 1 and 2 had less grassy smell of carrot than that of Test 3. Conventionally, Palatinose is added to mask the smell. It was shown that the Particles of Examples attain the masking effect like palatinose.

### Test Example 9: Production of Strawberry Jams and Evaluation

### Production of Strawberry Jams

Strawberry jams were produced, using the ingredients in the compositions of Tests 1 to 3 indicated in Table 15 below. A proportion of sucrose to the Particles of Experiment 4-2 was varied to obtain a strawberry jam having a Brix of 60 degrees and no crystals. In order to prevent formation of crystals, trehalulose syrup (Mildear75, Mitsui Sugar Co., Ltd.) was added in an amount of 20 parts by mass per total 100 parts by mass of the whole saccharides for each Test.

### Table 15

**Table 15: Ingredients and Compositions of Strawberry Jams**

| Composition (g) | Test 1 | Test 2 | Test 3 |
|---|---|---|---|
| Frozen Strawberry | **200** | **200** | **200** |
| Trehalulose Syrup | **20** | **20** | **20** |
| Sugar | **40** | **30** | **20** |
| Particles of Example 4-2 | **40** | **50** | **60** |

Crystals were formed in the jam of Test 3 after one-month storage in a refrigerator. No crystal was formed in the jams of Tests 1 and 2 after one-month storage in a refrigerator, nor even after freezed for three days, followed by thawing.

As to sweetness, the jam of Test 3 was not so sweet as that of Test 1, but was sufficiently sweet. The more amount of the Particles of Experiment 4-2 the jam contained, the lighter sweetness and the stronger sourness the jam had. The jam of Test 3 had good balance between the sweetness and the sourness, and gave a strong strawberry flavor.

### Test Example 10: Production of Chewing Gums and Evaluation

Chewing Gums were produced using Palatinose or the Particles of Example 4-1. A production method was as follows. First, 15 parts by mass of enzymatically-saccharified corn syrup (KosoSyrup H85C, Brix of 85 degrees, Nihon Cornstarch Corporation) was added to 30 parts by mass of a chewing gum base, and kneaded for 5 minutes by a kneader (Benchkneader PNV-1, IRIE SHOKAI Co., Ltd.) which was warmed at 45 degrees Celsius. To the resulting mixture, 54 parts by mass of Palatinose (powdery Palatinose ICP, Mitsui Sugar Co., Ltd.) or of the Particles of Example 4-1 was added in several portions and, then, 1 part by mass of glycerol (JUNSEI CHEMICAL CO., LTD., food additive grade) was added, and kneaded for 15 minutes. Then, 1 part by mass of a flavor (peppermint oil, TAKATA KORYO CO. , LTD.) was added to the mixture and, then, kneaded for 5 minutes . Then, the kneaded material was rolled into a stick gum (thickness of 2mm, width of 2 cm, length of 7 cm), using Palatinose (powdery Palatinose ICP, Mitsui Sugar Co., Ltd.) to prevent the material from skicking, wrapped with an aluminum foil to obtain a chewing gum.

The resulting gums were examined one week after the production. The chewing gum produced with the Particles of Example 4-1 was softer than that produced with Palatinose.

## Claims

1. A method for producing a solid material from a saccharide liquid comprising isomaltulose, wherein said saccharide liquid is obtained by allowing an alpha-glucosyltransferase to act on a sucrose liquid, wherein said method comprises steps:
- heating said saccharide liquid to adjust a solid content of said saccharide liquid to 77 to 96 mass%,
- subjecting a mixture resulting from the aforesaid step to a shearing force to generate crystal nuclei, while keeping a temperature of the product at 65 to 120 degrees Celsius, and
- cooling the mixture to obtain the solid material,
- wherein the solid material comprises isomaltulose of an amount of 70 to 90 mass% and trehalulose of an amount of 8 to 25 mass%, and is an aggregate of isomaltulose crystals and non-crystalline saccharide liquid which comprises trehalulose.

2. The method according to claim 1, wherein the adjustment of the solid content is done by heating said saccharide liquid at 110 to 120 degrees Celsius at normal pressure.

3. The method according to claim 1 or 2, wherein the non-crystalline saccharide liquid comprises trehalulose in a content of 40 to 99 mass% of the non-crystalline saccharide liquid.

4. The method according to anyone of claim 1 to 3, wherein the mass percentage of trehalulose, relative to the total mass of saccharides contained in the saccharide liquid, is 9 to 20 mass%, in particular 10 to 18 mass%.

5. The method according to anyone of claim 1 to 4, wherein the isomaltulose content of the solid material is 72 to 89 mass%, especially 74 to 88 mass%, even more particularly 75 to 85 mass%.

6. A solid material comprising isomaltulose of an amount of 70 to 90 mass%, especially 72 to 89 mass%, more especially 74 to 88 mass%, more especially 75 to 85 mass%, more especially more than 80 to 85 mass%, and trehalulose of an amount of 8 to 25 mass%, especially 9 to 20 mass%, more especially 10 to 18 mass%, wherein the solid material is an aggregate of isomaltulose crystals and non-crystalline saccharide liquid which comprises trehalulose and wherein a median diameter of the crystals is 0.1 to 20 µm, as determined by laser diffraction particle size distribution measurement.

7. The solid material according to claim 6, wherein a median diameter of the crystals is 0.2 to 15 µm, in particular 0.3 to 10 µ m.

8. The solid material according to claim 6 or 7, wherein the non-crystalline saccharide liquid comprises trehalulose in a content of 40 to 99 mass%, in particular 45 to 80 mass%, more particularly 50 to 70 mass%, of the mass of the non-crystalline saccharide liquid.

9. The solid material according to anyone of claims 6 to 8, wherein the water content of the solid material is 0.5 to 9 mass%, in particular 0.55 to 8 mass%, more especially 0.8 to 7 mass%.

10. The solid material according to any one of claims 6 to 9, wherein a color value is 600 or less in an aqueous solution of the solid material at a Brix of 25 degrees and an adjusted pH of 7, more preferably 500 or less, even more preferably 400 or less, 300 or less, or 200 or less, most preferably 100 or less.

11. The solid material according to any one of claims 6 to 10, wherein the solid material is in a form of particles and particles having a diameter of 5 mm or more, 4 mm or more, 3 mm or more, 2 mm or more, 1mm or more, or 600 µm or more, accounts for 1 mass% or less in a particle size distribution of the particles.

## Patentansprüche

1. Verfahren zur Herstellung eines festen Materials aus einer Isomaltulose umfassenden Saccharidflüssigkeit, wobei die Saccharidflüssigkeit erhalten wird, indem man eine alpha-Glucosyltransferase auf eine Saccharoseflüssigkeit einwirken lässt, wobei das Verfahren die folgenden Schritte umfasst:
- Erhitzen der Saccharidflüssigkeit, um einen Feststoffgehalt der Saccharidflüssigkeit auf 77 bis 96 Massen-% einzustellen,
- Unterziehen eines aus dem vorstehend genannten Schritt erhaltenen Gemischs einer Scherkraft, um Kristallkeime zu erzeugen, während eine Temperatur des Produkts bei 65 bis 120 Grad Celsius gehalten wird, und
- Abkühlen des Gemischs, um das feste Material zu erhalten,
- wobei das feste Material Isomaltulose in einer Menge von 70 bis 90 Massen-% und Trehalulose in einer Menge von 8 bis 25 Massen-% umfasst und ein Aggregat aus Isomaltulose-Kristallen und nicht kristalliner Saccharidflüssigkeit, die Trehalulose umfasst, ist.

2. Verfahren nach Anspruch 1, wobei die Einstellung des Feststoffgehalts durch Erhitzen der Saccharidflüssigkeit bei 110 bis 120 Grad Celsius unter Normaldruck erfolgt.

3. Verfahren nach Anspruch 1 oder 2, wobei die nicht kristalline Saccharidflüssigkeit Trehalulose in einem Gehalt von 40 bis 99 Massen-%, bezogen auf die Masse der nicht kristallinen Saccharidflüssigkeit, umfasst.

4. Verfahren nach einem von Anspruch 1 bis 3, wobei die Massenprozent Trehalulose, bezogen auf die Gesamtmasse der in der Saccharidflüssigkeit enthaltenen Saccharide, 9 bis 20 Massen-%, insbesondere 10 bis 18 Massen-% beträgt.

5. Verfahren nach einem von Anspruch 1 bis 4, wobei der Isomaltulose-Gehalt des festen Materials 72 bis 89 Massen-%, insbesondere 74 bis 88 Massen-%, noch spezieller 75 bis 85 Massen-% beträgt.

6. Festes Material, umfassend Isomaltulose in einer Menge von 70 bis 90 Massen-%, insbesondere 72 bis 89 Massen-%, spezieller 74 bis 88 Massen-%, spezieller 75 bis 85 Massen-%, spezieller mehr als 80 bis 85 Massen-%, und Trehalulose in einer Menge von 8 bis 25 Massen-%, insbesondere 9 bis 20 Massen-%, spezieller 10 bis 18 Massen-%, wobei das feste Material ein Aggregat aus Isomaltulose-Kristallen und nicht kristalliner Saccharidflüssigkeit, die Trehalulose umfasst, ist, und wobei ein mittlerer Durchmesser der Kristalle 0,1 bis 20 µm beträgt, wie mittels Laserbeugungspartikelgrößenverteilungsmessung bestimmt wird.

7. Festes Material nach Anspruch 6, wobei ein mittlerer Durchmesser der Kristalle 0,2 bis 15 µm, insbesondere 0,3 bis 10 µm beträgt.

8. Festes Material nach Anspruch 6 oder 7, wobei die nicht kristalline Saccharidflüssigkeit Trehalulose in einem Gehalt von 40 bis 99 Massen-%, insbesondere 45 bis 80 Massen-%, spezieller 50 bis 70 Massen-%, bezogen auf die Masse der nicht kristallinen Saccharidflüssigkeit, umfasst.

9. Festes Material nach einem der Ansprüche 6 bis 8, wobei der Wassergehalt des festen Materials 0,5 bis 9 Massen-%, insbesondere 0,55 bis 8 Massen-%, spezieller 0,8 bis 7 Massen-% beträgt.

10. Festes Material nach einem der Ansprüche 6 bis 9, wobei in einer wässrigen Lösung des festen Materials bei einem Brix von 25 Grad und einem eingestellten pH von 7 ein Farbwert 600 oder weniger, stärker bevorzugt 500 oder weniger, noch stärker bevorzugt 400 oder weniger, 300 oder weniger oder 200 oder weniger, am stärksten bevorzugt 100 oder weniger beträgt.

11. Festes Material nach einem der Ansprüche 6 bis 10, wobei das feste Material in Form von Partikeln vorliegt und Partikel mit einem Durchmesser von 5 mm oder mehr, 4 mm oder mehr, 3 mm oder mehr, 2 mm oder mehr, 1 mm oder mehr oder 600 µm oder mehr 1 Massen-% oder weniger in einer Partikelgrößenverteilung der Partikel ausmachen.

## Revendications

1. Procédé de production d'un matériau solide à partir d'un liquide à base de saccharide comprenant de l'isomaltulose, dans lequel ledit liquide à base de saccharide est obtenu en laissant agir une alpha-glucosyltransférase sur un liquide à base de saccharose, ledit procédé comprenant les étapes de :
- chauffage dudit liquide à base de saccharide pour ajuster une teneur en matières solides dudit liquide à base de saccharide à 77 à 96 % en masse,
- soumission d'un mélange résultant de l'étape mentionnée ci-dessus à une force de cisaillement pour générer des noyaux cristallins, tout en maintenant une température du produit de 65 à 120 degrés Celsius, et
- refroidissement du mélange pour obtenir le matériau solide,
- dans laquelle le matériau solide comprend de l'isomaltulose en une quantité de 70 à 90 % en masse et du tréhalulose en une quantité de 8 à 25 % en masse, et est un agrégat de cristaux d'isomaltulose et de liquide à base de saccharide non cristallin qui comprend du tréhalulose.

2. Procédé selon la revendication 1, dans lequel l'ajustement de la teneur en matières solides est effectué en chauffant ledit liquide à base de saccharide à 110 à 120 degrés Celsius à pression normale.

3. Procédé selon la revendication 1 ou 2, dans lequel le liquide à base de saccharide non cristallin comprend du tréhalulose à une teneur de 40 à 99 % en masse du liquide à base de saccharide non cristallin.

4. Procédé selon l'une quelconque des revendications 1 à 3, dans lequel le pourcentage en masse de tréhalulose, par rapport à la masse totale de saccharides contenus dans le liquide à base de saccharide, est de 9 à 20 % en masse, en particulier 10 à 18 % en masse.

5. Procédé selon l'une quelconque des revendications 1 à 4, dans lequel la teneur en isomaltulose du matériau solide est de 72 à 89 % en masse, en particulier 74 à 88 % en masse, encore plus particulièrement 75 à 85 % en masse.

6. Matériau solide comprenant de l'isomaltulose en une quantité de 70 à 90 % en masse, en particulier 72 à 89 % en masse, plus particulièrement 74 à 88 % en masse, plus particulièrement 75 à 85 % en masse, plus particulièrement plus de 80 à 85 % en masse, et du tréhalulose en une quantité de 8 à 25 % en masse, en particulier 9 à 20 % en masse, plus particulièrement 10 à 18 % en masse, le matériau solide étant un agrégat de cristaux d'isomaltulose et de liquide à base de saccharide non cristallin qui comprend du tréhalulose et dans lequel un diamètre médian des cristaux est de 0,1 à 20 um, tel que déterminé par mesure de distribution granulométrique par diffraction laser.

7. Matériau solide selon la revendication 6, dans lequel un diamètre médian des cristaux est de 0,2 à 15 µm, en particulier 0,3 à 10 µm.

8. Matériau solide selon la revendication 6 ou 7, dans lequel le liquide à base de saccharide non cristallin comprend du tréhalulose à une teneur de 40 à 99 % en masse, en particulier 45 à 80 % en masse, plus particulièrement 50 à 70 % en masse, de la masse du liquide à base de saccharide non cristallin.

9. Matériau solide selon l'une quelconque des revendications 6 à 8, dans lequel la teneur en eau du matériau solide est de 0,5 à 9 % en masse, en particulier de 0,55 à 8 % en masse, plus particulièrement de 0,8 à 7 % en masse.

10. Matériau solide selon l'une quelconque des revendications 6 à 9, dans lequel une valeur de couleur est de 600 ou moins dans une solution aqueuse du matériau solide à un indice Brix de 25 degrés et un pH ajusté de 7, plus préférablement 500 ou moins, encore plus préférablement 400 ou moins, 300 ou moins ou 200 ou moins, le plus préférablement 100 ou moins.

11. Matériau solide selon l'une quelconque des revendications 6 à 10, dans lequel le matériau solide est sous forme de particules et les particules ayant un diamètre de 5 mm ou plus, 4 mm ou plus, 3 mm ou plus, 2 mm ou plus , 1 mm ou plus, ou 600 um ou plus, représentent 1 % en masse ou moins dans une distribution granulométrique des particules.
